# EUROPEAN PATENT APPLICATION

(11) **EP 2 067 475 A1**
(43) Date of publication of application: **10.06.2009**
(21) Application number: 07807827.6
(22) Date of filing: 25.09.2007
(51) Int. Cl.: A61K 31/436, A61K 9/32, A61K 9/36, A61K 47/32, A61K 47/36, A61K 47/38

(54) **TACROLIMUS SUSTAINED-RELEASE PREPARATION**

(30) Priority: 26.09.2006 US 847087 P
(71) Applicant: Astellas Pharma Inc., Tokyo 103-8411 (JP)
(72) Inventor: KOJIMA, Hiroyuki, Tokyo 103-8411 (JP); KONDO, Hiromu, Tokyo 103-8411 (JP); YOSHIHARA, Keiichi, Tokyo 103-8411 (JP); TAKETANI, Yuko, Tokyo 103-8411 (JP); ISHII, Takuya, Tokyo 103-8411 (JP)
(74) Representative: Gille Hrabal Struck Neidlein Prop Roos
(86) International application number: PCT/JP2007/068555
(87) International publication number: WO 2008/041553

(57) **Abstract**

A controlled release dosage form of tacrolimus, comprising a solid dispersion of tacrolimus, wherein a controlled release base, which is selected from the group consisting of a water-soluble macromolecule, a gum base, and a membrane forming agent and does not form the solid dispersion of tacrolimus, is further contained, is disclosed. The controlled release dosage form of tacrolimus has an excellent controlled release and shows a stable blood concentration.

## Description

### TECHNICAL FIELD

The present invention relates to a controlled release dosage form of tacrolimus.

### BACKGROUND ART

Tacrolimus is a macrolide immunosuppressant and was discovered at a Lead Discovery Laboratories of Astellas Pharma Inc. (former name: Fujisawa Pharmaceutical. Co., Ltd.) in 1984. Due to its insolubility, tacrolimus must be treated to enhance the bioavailability thereof when orally administered, and a solid dispersion composition containing tacrolimus is known as such a treatment (patent reference 1).

Further, a controlled release dosage form of tacrolimus exhibiting a sufficiently sustained release and a high bioavailability when orally administered is known (patent reference 2). In this pharmaceutical preparation, insoluble tacrolimus is contained as a solid dispersion composition in which tacrolimus is present in an amorphous state in a solid base composed of ethyl cellulose (a water-insoluble base) and hydroxypropylmethyl cellulose (a water-soluble base). This can provide an excellent controlled release. However, although patent reference 2 discloses a use thereof as capsules, it does not explicitly disclose a use as tablets, and a controlled release dosage form as tablets is desired.

A pharmaceutical preparation comprising the solid dispersion composition containing tacrolimus is widely used as a potent immunosuppressant in transplantation, but has the problems of, for example, showing different profiles of a blood concentration of tacrolimus dependent on the time when the tacrolimus is administered, and an improvement is needed to achieve a stable blood concentration.

[patent reference 1] Japanese Unexamined Patent Publication (Kokai) No. 62-277321
[patent reference 2] WO 99/49863

### DISCLOSURE OF INVENTION

### PROBLEMS TO BE SOLVED BY THE INVENTION

An object of the present invention is to provide a controlled release dosage form of tacrolimus having an excellent controlled release, and a use of the controlled release dosage form showing a constant blood concentration profile.

### MEANS FOR SOLVING THE PROBLEMS

The present invention relates to:
[1] a controlled release dosage form of tacrolimus, comprising a solid dispersion of tacrolimus, wherein a controlled release base, which is selected from the group consisting of a water-soluble macromolecule, a gum base, and a membrane forming agent and does not form the solid dispersion of tacrolimus, is further contained,
[2] the controlled release dosage form of tacrolimus of [1], wherein the form is a tablet,
[3] the controlled release dosage form of tacrolimus of [1], comprising the solid dispersion of tacrolimus and the water-soluble macromolecule, wherein a % dissolved after 4 hours from the beginning of a dissolution test is 35% to 85%,
[4] the controlled release dosage form of tacrolimus of [3], wherein the water-soluble macromolecule is selected from the group consisting of hydroxypropylmethyl cellulose, hydroxypropylmethyl cellulose phthalate, hydroxymethyl cellulose, methyl cellulose, hydroxyethyl cellulose, hydroxypropyl cellulose, polyvinylpyrrolidone, polyvinyl alcohol, a carboxyvinyl polymer, and carboxymethyl cellulose sodium,
[5] the controlled release dosage form of tacrolimus of [1], comprising the solid dispersion of tacrolimus and the gum base, wherein a % dissolved after 4 hours from the beginning of a dissolution test is 35% to 85%,
[6] the controlled release dosage form of tacrolimus of [5], wherein the gum base is a combination of a heteropolysaccharide and a homopolysaccharide,
[7] the controlled release dosage form of tacrolimus of [6], wherein the heteropolysaccharide is xanthan gum and the homopolysaccharide is locust bean gum,
[8] the controlled release dosage form of tacrolimus of [6] or [7], further comprising calcium sulfate and/or a water-soluble base,
[9] the controlled release dosage form of tacrolimus [8], wherein the water-soluble base is selected from the group consisting of dextrose, sucrose, fructose, maltose, xylitol, and citric acid,
[10] the controlled release dosage form of tacrolimus of [1], wherein an uncoated tablet or granule containing the solid dispersion of tacrolimus is coated with a coating liquid containing a membrane forming agent, and a % dissolved after 4 hours from the beginning of a dissolution test is 35% to 85%,
[11] the controlled release dosage form of tacrolimus of [10], wherein the membrane forming agent is a water-insoluble macromolecule,
[12] the controlled release dosage form of tacrolimus of [11], wherein the water-insoluble macromolecule is selected from the group consisting of an aminoalkylmethacrylate copolymer, a methacrylate copolymer, ethyl cellulose, and cellulose acetate,
[13] the controlled release dosage form of tacrolimus of [11] or [12], wherein the coating liquid further contains a water-soluble base and/or a water-soluble macromolecule,
[14] the controlled release dosage form of tacrolimus of [13], wherein the water-insoluble macromolecule is selected from the group consisting of an aminoalkylmethacrylate copolymer, a methacrylate copolymer, ethyl cellulose, and cellulose acetate, the water-soluble base is selected from the group consisting of dextrose, sucrose, fructose, maltose, xylitol, citric acid, sodium chloride, and polyoxyethylene sorbitan monooleate, and the water-soluble macromolecule is selected from the group consisting of hydroxypropylmethyl cellulose, hydroxypropyl cellulose, polyvinylpyrrolidone, polyethylene glycol, and polyvinyl alcohol,
[15] the controlled release dosage form of tacrolimus of [1], comprising
   (1) a first layer containing 5% to 90% of the water-soluble macromolecule, and
   (2) a second layer containing the solid dispersion of tacrolimus, and the water-soluble macromolecule which does not form the solid dispersion of tacrolimus,
   wherein a % dissolved after 4 hours from the beginning of a dissolution test is 35% to 85%,
[16] the controlled release dosage form of tacrolimus of [15], further comprising a third layer containing a water-soluble macromolecule,
[17] the controlled release dosage form of tacrolimus of [15] or [16], wherein the water-soluble macromolecule is selected from the group consisting of hydroxypropylmethyl cellulose, hydroxypropyl cellulose, hydroxymethyl cellulose, and hydroxyethyl cellulose,
[18] the controlled release dosage form of tacrolimus of [15] to [17], further comprising microcrystalline cellulose in the second layer,
[19] the controlled release dosage form of tacrolimus of [15] to [18], further comprising a water-soluble base selected from the group consisting of dextrose, sucrose, fructose, maltose, xylitol, citric acid, lactose, and mannitol in the second layer,
[20] use of a controlled release dosage form of tacrolimus for reducing an influence caused by a time when tacrolimus is administered to a human,
[21] use of the controlled release dosage form of tacrolimus of [20], wherein a ratio (b/a) of a maximum blood drug concentration (b) when administered in the evening to a maximum blood drug concentration (a) when administered in the morning is 0.5 or more,
[22] use of the controlled release dosage form of tacrolimus of [20] or [21], wherein a % dissolved after 4 hours from the beginning of a dissolution test is 35% to 85%,
[23] use of the controlled release dosage form of tacrolimus of [20] to [22], comprising the solid dispersion wherein tacrolimus is present in an amorphous state in a solid base composed of ethyl cellulose and hydroxypropylmethyl cellulose, and
[24] use of the controlled release dosage form of tacrolimus of [20] to [23], wherein the controlled release dosage form of tacrolimus contains a solid dispersion of tacrolimus, and a controlled release base which is selected from the group consisting of a water-soluble macromolecule, a gum base, and a membrane forming agent and does not form the solid dispersion of tacrolimus.
The term "% dissolved" as used herein means a percentage of dissolution calculated in accordance with a dissolution test, method 2 (paddle method), described in the Japanese Pharmacopoeia. More particularly, 900 mL of a solution prepared by dissolving 0.005% of hydroxypropyl cellulose (HPC) in the second fluid (JP2) of the disintegration test described in the Japanese Pharmacopoeia is used as a test medium, and a dissolution of a test sample is carried out at 37°C without the use of a sinker, at a paddle rotation speed of 100 rpm.

### EFFECTS OF THE INVENTION

According to the present invention, a controlled release dosage form of tacrolimus showing an excellent controlled release and a constant blood concentration profile can be provided.

### BEST MODE FOR CARRYING OUT THE INVENTION

The controlled release dosage form of tacrolimus according to the present invention contains a solid dispersion composition of tacrolimus, and one or more controlled release bases, which are selected from the group consisting of a water-soluble macromolecle, a gum base, and a membrane forming agent and do not form the solid dispersion composition. The controlled release dosage form of tacrolimus according to the present invention shows a % dissolved of 35% to 85%, preferably 35% to 65%, after 4 hours from the beginning of a dissolution test.

Tacrolimus [chemical name: 17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone], which is the active ingredient of the solid dispersion of tacrolimus used in the present invention, may be obtained by isolation and purification from a culture of Streptomyces tsukubaensis, for example, in accordance with a method disclosed in Japanese Unexamined Patent Publication (Kokai) No. 62-277321.

As the solid dispersion of tacrolimus, for example, a tacrolimus-containing composition disclosed in Japanese Unexamined Patent Publication (Kokai) No. 62-277321 may be exemplified. This tacrolimus-containing composition contains tacrolimus and a water-soluble polymer (which is synonymous with a water-soluble macromolecule described below), and, if desired, may further contain various additives which are conventionally used in the field of pharmaceutical preparations, such as a filler, a disintegrator, a coloring agent, a sweetener, a flavor, a diluent, or a lubricant.

The solid dispersion of tacrolimus used in the present invention may be prepared in accordance with, for example, a method disclosed in Japanese Unexamined Patent Publication (Kokai) No. 62-277321. More particularly, the solid dispersion of tacrolimus may be prepared by dissolving tacrolimus in an organic solvent (for example, a lower alcohol, such as methanol, ethanol, propanol, isopropanaol, or the like, ethyl acetate, or diethyl ether), adding a water-soluble polymer, adding one or more additives to the resulting suspension or solution if desired, and removing the organic solvent from the mixture.

The water-soluble polymer is not particularly limited, so long as tacrolimus may be dispersed. As the water-soluble polymer, there may be mentioned, for example, a water-soluble cellulose polymer, such as hydroxypropylmethyl cellulose, hydroxypropyl cellulose, methyl cellulose, or the like. The content of the water-soluble polymer is not particularly limited, so long as tacrolimus may be dispersed, and is preferably 0.1:1 to 20:1 as a ratio by weight of the water-soluble polymer to tacrolimus (water-soluble polymer:tacrolimus).

As a preferred base contained in the solid dispersion of tacrolimus used in the present invention (hereinafter referred to as a solid base), a water-insoluble base may be exemplified. For example, a solid dispersion in which tacrolimus is present in an amorphous state in a solid base composed of ethyl cellulose and hydroxypropylmethyl cellulose may be prepared in accordance with a method disclosed in WO 99/49863. Further, an aminoalkylmethacrylate copolymer E such as Eudragit E (product name; degussa) may be used as a base for the solid dispersion. Furthermore, as other solid dispersions, there may be mentioned, for example, a solid dispersion which contains various macromolecular bases and is obtained by a twin screw extruder (WO 2003/077827), a solid dispersion with a water-insoluble macromolecule such as a biodegradable macromolecule (WO 2003/043603, WO 2004/000279, or WO 2004/071494), a solid dispersion with a lipid (WO 2003/013566, WO 2004/009075, WO 2004/087052, WO 98/40051, or WO 99/00113), a solid dispersion with a self-emulsifying agent (WO 2005/030169), a solid dispersion with an extract (WO 2003/049753, WO 2004/009061, or WO 2004/067018), a solid dispersion obtained by solubilization with phospholipid micelle (WO 99/44642), a solid dispersion obtained by spray drying with an O/W emulsion (WO 2004/062643), or the like.

The controlled release dosage form of tacrolimus according to the present invention includes, for example,
(1) a matrix type pharmaceutical preparation utilizing a water-soluble macromolecule,
(2) a controlled release pharmaceutical preparation with a coating membrane,
(3) a multilayered pharmaceutical preparation consisting of a drug core and a release-controlling layer, which are geometrically arranged, and
(4) a gel pharmaceutical preparation in which plural gums are combined.

### (1) Matrix type pharmaceutical preparation utilizing water-soluble macromolecule

A matrix type pharmaceutical preparation utilizing a water-soluble macromolecule, an embodiment of the controlled release dosage form of tacrolimus according to the present invention, is a controlled release dosage form in which the drug is homogenously dispersed in one or more water-soluble macromolecules, such as hydroxypropylmethyl cellulose (HPMC).

Techniques for obtaining such a matrix type pharmaceutical preparation which may be used in the controlled release dosage form of tacrolimus according to the present invention are disclosed, for example, in WO 93/16686, the contents of which are incorporated herein by reference.

When hydroxypropylmethyl cellulose, a water-soluble macromolecule, is brought into contact with water, hydration thereof is caused, and a hydrogel layer is formed on the surface of a matrix. This gel layer containing a drug formed on the matrix surface is gradually dissolved and eroded, to release the drug from the layer. The matrix type pharmaceutical preparation of the present invention is characterized in that a drug may be controllably released by repeating the contact with water, the formation of the gel layer containing the drug, and the dissolution and erosion of the gel layer.

The matrix type pharmaceutical preparation of the present invention is characterized in that a controlled release filler consisting of a water-soluble macromolecule, an inactive diluent, and a physiologically active substance are homogenously dispersed. The water-soluble macromolecule is not particularly limited, so long as it is gradually gelled, eroded, dissolved, and/or disintegrated when exposed to an environmental fluid. The water-soluble macromolecules include, for example, a gastro-solbule macromolecule or an enteric macromolecule, the water solubility of which is dependent on a pH adjustment, an ion strength, or the like. As the water-soluble macromolecules, there may be mentioned, for example, hydroxypropylmethyl cellulose having a molecular weight of 1,000 to 4,000,000, hydroxymethyl cellulose, hydroxyethyl cellulose, hydroxypropyl cellulose having a molecular weight of 2,000 to 2,000,000, hydroxypropylmethyl cellulose phthalate having a labeled viscosity of 30 to 200 mm²/s [at 20°C; a 10% solution prepared by dissolving hydroxypropylmethyl cellulose phthalate in a methanol/dichloromethane mixture (1:1)], carboxyvinyl polymers, chitosans, mannans, galactomannans, xanthans, carageenans, amylose, alginic acid, salts and derivatives thereof, pectin, acrylates, aminoalkylmethacrylate copolymers, methacrylate copolymers, polyacid anhydrides, polyamino acids, poly(methylvinyl ether/maleic anhydride)polymers, polyvinyl alcohols, polyvinylpyrrolidone, glucans, scleroglucans, carboxymethyl cellulose sodium and derivatives thereof, methyl cellulose, or conventional water-soluble cellulose derivatives. Hydroxypropylmethyl cellulose having a molecular weight of 1,000 to 2,000,000, or carboxyvinyl polymers of 3,000 to 45,000 cps (at 25°C; a 0.5% aqueous solution) is preferable, and hydroxypropylmethyl cellulose having a molecular weight of 10,000 to 1,000,000, or carboxyvinyl polymers of 4,000 to 40,000 cps (at 2.5 °C; a 0.5% aqueous solution) is more preferable. The content of the water-soluble macromolecule is 10 W/W% or more per pharmaceutical preparation unit, preferably 30 W/W% or more, more preferably 70 W/W% or more. These water-soluble molecules may be contained alone or as a combination thereof in an appropriate amount(s).

Various fillers for medicaments may be appropriately used to prepare the matrix type pharmaceutical preparation of the present invention. The fillers for medicaments are not particularly limited, so long as they are pharmaceutically acceptable and may be used as additives for medicament. As the fillers, for example, a diluent, a binder, a disintegrator, an acidulant, an effervescent agent, an artificial sweetener, a flavor, a lubricant, a coloring agent, or the like may be used. The diluent may be selected from the group consisting of mannitol, lactose, starches derived from various organs, sorbitol, xylitol, citric acid, microcrystalline cellulose, and/or a diluent capable of generally promoting a penetration of water or an aqueous liquid into a pharmaceutical preparation. The binders include, for example, hydroxypropylmethyl cellulose, hydroxypropyl cellulose, polyvinyl alcohol, methyl cellulose, gum arabic, and the like. The disintegrators include, for example, a corn starch, a starch, carmellose calcium, carmellose sodium, low-substituted hydroxypropyl cellulose, and the like. The acidulants include, for example, citric acid, tartaric acid, malic acid, and the like. The effervescent agents include, for example, sodium bicarbonate and the like. The artificial sweeteners include, for example, saccharin sodium, dipotassium glycyrrhizinate, aspartame, stevia, thaumatin, and the like. The flavors include, for example, lemon, lemon-lime, orange, menthol, and the like. The lubricants include, for example, magnesium stearate, calcium stearate, sucrose fatty acid esters, polyethylene glycol, talc, stearic acid, and the like. These fillers for medicaments may be contained alone or as a combination thereof in an appropriate amount (s).

The matrix type pharmaceutical preparation of the present invention may be manufactured by a known method per se. In particular, tablets may be manufactured by a tablet forming method which is commonly used and known to those skilled in the art. The tabletting pressure is generally within a range of 3 to 20 kN. In a small scale, tablets may be prepared, in accordance with methods explained in detail in the following Examples, by preparing powder and/or granules with a mortar and a pestle, and forming the powder and/or granules into tablets by using an oil press tabletting machine.

### (2) Controlled release pharmaceutical preparation with coating membrane

As a method for controlling the release (i.e., controlled release) of a drug from a pharmaceutical preparation, a coating membrane is applied to the surface of a pharmaceutical preparation by coating. The kind of coating membrane is not particularly limited. The coating may be applied to not only a shaped preparation such as a tablet or the like, but also various preparations such as powder, granules, pellets, or the like.

A coating liquid may contain, for example, a membrane forming agent (mainly a macromolecule), a plasticizer (which provides plasticity, flexibility, and extensibility to a coating membrane), a water-soluble base (such as lactose, sodium chloride, or the like), a dispersing agent (which prevents particles or tablets from adhering and aggregating after the coating), or the like. These components may be dissolved or dispersed in an appropriate solvent, such as water, alcohol, or the like, to prepare the coating liquid.

The release of a drug from the pharmaceutical preparation can be controlled by appropriately adjusting, for example, the kinds and the mixing ratio of components contained in the coating liquid, an amount of coating, or the like. For example, a preferable ratio of the membrane forming agent to the water-soluble base is 99:1 to 50:50 (membrane forming agent: water-soluble base). The content of the coating membrane is preferably approximately 2 to 30 parts by weight, with respect to 100 parts by weight of an uncoated tablet.

As a coating method, there may be mentioned, for example, a method in which a coating liquid, such as an organic solvent solution, or a mixing solution or suspension of an organic solvent and water, is sprayed while being rotated, by using a coating pan, or a method in which a coating liquid is sprayed while being fluidized by air blown from the bottom of a fluidized bed. Further, a coating liquid prepared by dissolving or dispersing a membrane forming agent in a solvent may be sprayed, and then the solvent may be removed by drying to form a coating membrane on the surface of a pharmaceutical preparation. As a simple method, a coating membrane may be formed by immersing shaped preparations or the like in a coating liquid.

As the membrane forming agent as used herein, there may be mentioned, for example, a water-insoluble macromolecule or a water-soluble macromolecule. The membrane forming agent is not particularly limited, so long as it is pharmaceutically acceptable and biocompatible. These membrane forming agents may be added alone or as a combination thereof in an appropriate amount(s).
As the water-insoluble macromolecule, there may be mentioned, for example, dibenzyl phthalate, dihexyl phthalate, butyl octyl phthalate, beeswax, carnauba wax, cetyl alchohol, cetyl stearyl alchohol, glyceryl behenate, lipids, fats, resins such as shellac or the like, cellulose derivatives such as ethyl cellulose, cellulose acetate, or the like, polyacrylate derivatives such as aminoacrylmethacryl copolymer (product name: Eudragit RS) or the like, polymethacrylate derivatives such as methacrylate copolymer (product name: Eudragit L) or the like, hydroxypropylmethyl cellulose acetate succinate, polylactic acid, polyglycolic acid, or the like.
As the water-soluble macromolecule, there may be mentioned, for example, hydroxypropylmethyl cellulose, hydroxypropyl cellulose, hydroxyethyl cellulose, carmellose sodium, methyl cellulose, polyvinylpyrrolidone, polyethylene glycol, polyvinyl alcohol, or the like.

To enhance a hydrophilic property of the coating membrane, a water-soluble base may be added. As the water-soluble base, there may be mentioned, for example, maltose, sucrose, lactose, sodium chloride, citric acid, polyethylene glycol 400, dextrose, fructose, xylitol, polyoxyethylene sorbitan monooleate, or the like.
The coating liquid which may be used in the present invention preferably contains one or more of the above-mentioned water-insoluble macromolecules, and more preferably further contains one or more of the water-soluble macromolecules and/or one or more of the water-soluble bases.

Further, the coating liquid may contain a plasticizer to provide plasticity, flexibility, and extensibility to the coating membrane. As the plasticizer, there may be mentioned, for example, triacetin, dioctyl azelate, epoxidized tallate, triisooctyl trimellitate, triisononyl trimellitate, sucrose acetate isobutyrate, soybean oil, propylene glycol, glycerol, polyethylene glycol, glycerol triacetate (triacetin), triethyl citrate, acetyl triethyl citrate, diethyl phthalate, diethyl sebacate, dibutyl sebacate, acetylated monoglyceride, castor oil, liquid paraffin, or the like.

If desired, a surfactant and/or a disintegrator may be added. As such a surfactant which may be used in the coating membrane, a surfactant having an HLB value of approximately 10 to 25, such as polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40-stearate, sodium oleate, or the like, may be used.
As the disintegrator, there may be mentioned, for example, starches, clay, cellulose, algin, gums, crosslinked starches, cellulose, or polymers. Typically, for example, corn starch, potato starch, croscarmellose, crospovidone, sodium starch glycorate, Veegum HV, methyl cellulose, agar, bentonite, carboxyl methyl cellulose, alginic acid, guar gum, or the like, may be used.

As a solvent suitable for manufacturing the pharmaceutical preparation of the present invention, an aqueous or inert organic solvent which does not adversely affect substances used in the system may be used. As the solvent, there may be mentioned, for example, aqueous solvents, alcohols, ketones, esters, ethers, aliphatic hydrocarbons, halogenated solvents, cycloaliphatic, aromatic, or heterocyclic solvents, or a mixture thereof. Typical solvents may be, for example, acetone, diacetone alcohol, methanol, ethanol, isopropanol, butanol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride, nitroethane, nitropropane, tetrachloroethane, ethyl ether, isopropyl ether, cyclohexane, cyclooctane, benzene, toluene, naphtha, 1,4-dioxane, tetrahydrofuran, diglyme, water, an aqueous solvent containing an inorganic salt such as sodium chloride, calcium chloride, or the like, or a mixture thereof, such as a mixture of acetone and water, a mixture of acetone and methanol, a mixture of acetone and ethanol, a mixture of methylene dichloride and methanol, or a mixture of ethylene dichloride and methanol.

### (3) Multilayered pharmaceutical preparation consisting of drug core and release-controlling layer which are geometrically arranged

A multilayered pharmaceutical preparation, an embodiment of the controlled release dosage form of tacrolimus according to the present invention, may be a two-layered or three-layered controlled release dosage form, characterized by consisting of a drug-containing layer and a release-controlling layer, and consisting of:
a) the first layer (layer 1) which is prepared by compressing a mixture or granules containing 5 to 90 W/W% (preferably 10 to 85 W/W%) of a water-soluble molecule in this layer, and has a property of being swollen by contact with environmental fluids,
b) the second layer (layer 2) comprising tacrolimus or a pharmaceutically acceptable salt thereof, a water-soluble macromolecule, and other filler(s), which is adjacent to the first layer, has a property suitable to compression-molding, and is designed to release the physiologically active substance within a predetermined period of time, and
c) the third layer (layer 3) (which may be optionally added to the layer 2) which contains a water-soluble mactromolecule capable of being generally gelled and/or swollen followed by optionally being disintegrated, and has a property of controlling the release of tacrolimus or a pharmaceutically acceptable salt thereof from the layer 2. The "environmental fluids" include, for example, an aqueous solution as used in a dissolution test, as well as body fluids such as blood or gastrointestinal fluids.

Techniques for such a multilayered pharmaceutical preparation which may be used in the controlled release dosage form of tacrolimus according to the present invention are disclosed in, for example, U.S. Patent No. 4,839,177, U.S. Patent No. 5,422,123, U.S. Patent No. 5,780,057, U.S. Patent No. 6,149,940, Japanese Unexamined Patent Publication (Kokai) No. 2005-162736, and Japanese Unexamined Patent Publication (Kokai) No. 2005-162737, the contents of which are incorporated herein by reference. As disclosed in U.S. Patent No. 4,839,177 and U.S. Patent No. 5,422,123, the multilayered pharmaceutical preparation is characterized in that a release rate of the drug from the pharmaceutical preparation is controlled by sandwiching the layer 2 containing the drug between the layer 1 and the layer 3 in which the drug is not contained or is optionally contained. Further, as disclosed in U.S. Patent No. 5,780,057 and U.S. Patent No. 6,149,940, it is known that when the multilayered pharmaceutical preparation is brought into contact with body fluids, at least one of the layer 1 and the layer 3 are rapidly swollen followed by the layer 2 is swollen, that is, the volume of the pharmaceutical preparation is significantly increased, and as a result, the pharmaceutical preparation remains in the stomach for a longer period of time, and almost all of the active substance contained therein is released and absorbed at the upper gastrointestinal tract in a controlled manner.

The layer 1 and the layer 3 may have the same composition and the same functional properties, or may have different compositions and different functional properties. When the layer 1 and the layer 3 have the same composition and functional properties, the amounts and thicknesses of the layers 1 and 3 which sandwich the layer 2 may be changed. At least one of the layers 1 and 3 acts as a barrier for the release of the active substance, that is, it is impermeable enough for tacrolimus or a salt thereof contained in the layer 2 not to be released or diffused therefrom. Further, at least one of the layers 1 and 3 can be rapidly swollen, that is, the volume thereof is rapidly increased. The layer 3 may optionally contain the drug so that a drug release which is different from that released from the layer 2 can be supplementally added to the pharmaceutical preparation.

The water-soluble macromolecules used in the layer 1, the layer 3, and the layer 2 are not particularly limited, so long as they are pharmaceutically acceptable and biocompatible. Such water-soluble macromolecule may be gradually dissolved and/or gelled in an aqueous liquid, and/or may be gelled rapidly or at a different rate and then optionally disintegrated. As the water-soluble macromolecules, there may be mentioned, for example, hydroxymethyl cellulose, hydroxyethyl cellulose, hydroxypropylmethyl cellulose having a molecular weight of 1,000 to 4,000,000, hydroxypropyl cellulose having a molecular weight of 2,000 to 2,000,000, carboxyvinyl polymers, chitosans, mannans, galactomannans, xanthans, carageenans, amylose, alginic acid, salts and derivatives thereof, pectin, acrylates, methacrylate, acrylate/methacrylate copolymers, polyacid anhydrides, polyamino acids, poly(methylvinyl ether/maleic anhydride)polymers, polyvinyl alcohols, glucans, scleroglucans, carboxymethyl cellulose sodium and derivatives thereof, ethyl cellulose, methyl cellulose, or conventional water-soluble cellulose derivatives. Hydroxypropylmethyl cellulose having a molecular weight of 3,000 to 2,000,000 is preferable. The content of the water-soluble macromolecule in the layer 1 or the layer 3 is generally 5 to 90 W/W%, preferably 10 to 85 W/W%, more preferably 20 to 80 W/W%, with respect to the weight of each layer. The content of the water-soluble macromolecule in the layer 2 is generally 5 to 90 W/W%, preferably 10 to 85 W/W%, to the weight of the layer.

In the process for preparing the layer 1 and the layer 3, a water-soluble filler which promotes the degree of wetness of the layers may be used, to rapidly increase the volume of the multilayerd pharmaceutical preparation containing the above water-soluble macromolecule. The water-soluble filler may be preferably selected from a group of fillers having an extremely rapid disintegrability, such as cross-linked polyvinylpyrrolidone, hydroxypropyl cellulose or hydroxypropylmethyl cellulose having a low or medium molecular weight, cross-linked carboxymethyl cellulose sodium, carboxymethyl starch or salts thereof, divinylbenzene/potassium methacrylate copolymers, or the like.
The content of the filler is 1 to 90 W/W% or less, preferably 5 to 50 W/W% of each layer.

If desired, a surfactant (anionic, cationic, or nonionic surfactants) may be further used to improve the degree of wetness. As a result, tablets and environmental fluids may conform with each other more rapidly, and the tablets, particularly the gel-forming layer, may be gelled more rapidly. As the surfactant, there may be mentioned, for example, sodium laurylsulfate, sodium ricinolate, sodium tetradecylsulfonate, sodium dioctylsulfosuccinate, cetomagrogol, poloxamer, glycerol monostearate, polysorbate, sorbitan monolaurate, lecithins, or other pharmaceutically acceptable surfactants.

If desired, another substance which modifies hydration may be further used. Such a substance may be selected from, for example, mannitol, lactose, starches derived from various organs, sorbitol, xylitol, microcrystalline cellulose, and/or a diluent capable of generally promoting a penetration of water or an aqueous liquid into a pharmaceutical composition; or a hydrophobic diluent to retard a penetration of water or an aqueous liquid into a pharmaceutical preparation, such as ethyl cellulose, glycerol monostearate, palmitate, or hydrogenated or non-hydrogenated vegetable oils (for example, hydrogenated castor oil, wax, monoglyceride, diglyceride, or triglyceride). It is preferable to select ethyl cellulose or hydrogenated vegetable oils as the hydrophobic diluent.
The content of the hydrophobic diluent in the layer 1 or the layer 3 is generally 1 to 60 W/W%, preferably 5 to 40 W/W%, more preferably 10 to 30 W/W%, with respect to the weight of each layer.
To control the release rate of tacrolimus from the pharmaceutical preparation, microcrystalline or a water-soluble base, such as dextrose, sucrose, fructose, maltose, xylitol, citric acid, lactose, mannitol, or the like, may be used in the layer 2, if desired.
The content of microcrystalline and/or the water-soluble base in the layer 2 is generally 5 to 90 W/W%, preferably 10 to 80 W/W%, more preferably 20 to 70 W/W%, with respect to the weight of the layer.

The multilayered pharmaceutical preparation of the present invention may contain, for example, a lubricant, such as magnesium stearate, talc, stearic acid, glycerol monostearate, polyoxyethylene glycol having a molecular weight of 400 to 7,000,000, hydrogenated castor oil, glycerol behenate, monoglyceride, diglyceride, triglyceride, or the like, a fluidizing agent such as colloidal silica or other silica, a binder, a buffer, an absorbing agent, or other pharmaceutically acceptable additives.

The multilayered pharmaceutical preparation of the present invention may be manufactured, for example, by mixing powder and/or granules by a known manufacturing technique per se, and forming the mixture into tablets by compression. A two-layered or three-layered pharmaceutical preparation, such as a tablet, may be manufactured by a known method per se. The multilayered pharmaceutical preparation of the present invention may be manufactured, for example, by using a rotary press capable of manufacturing multilayered tablets. It is preferable that a tabletting pressure is generally 7 to 50 kN. When the tablets are manufactured on a small scale, a mortar and pestle may be used to prepare powder and/or granules, and then, an oil press tabletting machine may be used to manufacture two-layered or three-layered tablets. The thickness of each layer of the pharmaceutical preparation may vary according to the content of the active substance, and is preferably 0.2 to 8 mm, more preferably 1 to 4 mm.
In the pharmaceutical preparation of the present invention, for example, a coating layer with a macromolecular material may be applied to the pharmaceutical composition. Such a coating may be applied by using an organic or aqueous solution, in accordance with a known method per se.

When the multilayered pharmaceutical preparation of the present invention is brought into contact with gastric juices in the gastrointestinal tract and/or liquids, the volume thereof is rapidly increased. This increase in volume may be determined and limited in a single layer or several layers of the pharmaceutical preparation. Such a pharmaceutical preparation may be in a form of a tablet, small tablets, or a gelatin capsule consisting of small tablets. Further, at least two small tablets may be combined in the same pharmaceutical preparation, and may be packed in, for example, a wafer capsule or a gelatin capsule. When the pharmaceutical preparation consists of small tablets, each small tablet may have a different composition or the same composition.

### (4) Gel pharmaceutical preparation in which plural gums are combined

A gel pharmaceutical preparation in which plural gums are combined, an embodiment of the controlled release dosage form of tacrolimus according to the present invention, is characterized by comprising at least the solid dispersion of tacrolimus and a gum base. The gum base as used herein means a controlled release filler comprising a homopolysaccharide which can form a crosslinkage with a heteropolysaccharide gum when exposed to the heteropolysaccharide gum and environmental fluids (such as body fluids, an aqueous solution for an in vitro dissolution test, or the like). The controlled release filler may further comprise a water-soluble cationic crosslinking agent and/or a water-soluble base, to enhance a gel strength and achieve an excellent controlled release. As the water-soluble cationic crosslinking agent, calcium sulfate or the like may be used. The gel pharmaceutical preparation may further contain a commonly used filler.

Techniques for obtaining the gel pharmaceutical preparation in which plural gums are combined, which may be used in the controlled release dosage form of tacrolimus according to the present invention, are disclosed in, for example, U.S. Patent No. 4,994,276, U.S. Patent No. 5,128,143, U.S. Patent No. 5,135,757, and Japanese Patent No. 2832248. As disclosed therein, it is known that a heterogeneously dispersed filler comprising a combination of a heteropolysaccharide and a homopolysaccharide exhibiting a synergistic effect, such as a combination of two or more polysaccharide gums, has a viscosity higher than that of any single gum, and can cause a rapid hydration, and thus a harder gel is generated more rapidly. The contents of the above patent references are incorporated herein by reference.

The heteropolysaccharide as used herein is defined as a water-soluble polysaccharide containing two or more sugar units. The heteropolysaccharide is not particularly limited, so long as it has a branched-chain or spiral configuration, and has an excellent water absorbing property and a high viscosity improving property. As the heteropolysaccharide, for example, xanthan gum or derivatives thereof (such as deacylated xanthan gum), carboxymethyl ether, or propylene glycol ester are preferable, and xanthan gum having a high molecular weight (>10⁶) is more preferable.

The homopolysaccharide as used herein is not particularly limited, so long as it is a polysaccharide consisting of mannose and galactose only, and can form a crosslinkage with a heteropolysaccharide. Locust bean gum having a high ratio of mannose to galactose is more preferable than other galactomannans such as guar or hydroxypropyl guar.
Other naturally-occurring polysaccharide gums may be used in the present invention. As such polysaccharides, there may be mentioned, for example, alginic acid derivatives, carrageenan, tragacanth gum, gum parable, karaya gum, polyethylene glycol esters of these gums, chitin, chitosan, mucopolysaccharide, konjak, starch, substituted starch, starch fragment, dextrin, British gum having a molecular weight of approximately 10,000 Da, dextran, or the like. The starch may be used in an unmodified form, for example, an ungelled starch such as potato, rice, banana, or the like, or a semisynthetic or gelled starch.

As a combination of the heteropolysaccharide and the homopolysaccharide, the combination of xanthan gum and locust bean gum is particularly preferable. The content ratio of the heteropolysaccharide and the homopolysaccharide is not particularly limited, so long as it is an amount effective in enhancing a desired gel strength. Such a ratio (heteropolysaccharide gum: galactomannan) is approximately 3:1 to approximately 1:3, preferably approximately 1:1.

The water-soluble cationic crosslinking agent as used herein is not particularly limited, so long as it is a pharmaceutically acceptable binder having a monovalent or polyvalent metal cation. As the binder, for example, calcium sulfate or the like may be used.

The water-soluble base as used herein is not particularly limited, so long as it is pharmaceutically acceptable. As the water-soluble base, there may be mentioned, for example, dextrose, sucrose, fructose, maltose, xylitol, citric acid, or the like.

The gel pharmaceutical preparation in which plural gums are combined of the present invention may be manufactured, for example, in a pharmaceutically acceptable form for oral administration such as a tablet or the like. In an embodiment, (1) a heteropolysaccharide gum, and a homopolysaccharide which can form a crosslinkage with the heteropolysaccharide gum when exposed to environmental fluids are mixed together under the dry condition with a pharmaceutically acceptable water-soluble base in a desired ratio, (2) the resulting mixture is subject to a wet granulation, (3) the granules are dried, (4) the dried granules are pulverized to obtain a controlled release filler having a desired particle size, (5) the resulting controlled release filler is granulated together with tacrolimus or a pharmaceutical acceptable salt thereof, (6) the resulting granules are dried, (7) a conventional filler, such as a lubricant or the like, is added thereto, and (8) the resulting mixture is formed by compression into, for example, tablets. In another embodiment, a mixture of the controlled release filler and tacrolimus or a pharmaceutical acceptable salt thereof may be granulated, together with an a solution of a hydrophobic substance (such as ethyl cellulose or the like) in an amount sufficient to retard the hydration of the filler (i.e., gums) without the destruction thereof, and then a conventional filler such as a lubricant is added thereto, and the resulting mixture is formed by compression into, for example, tablets.
In the wet granulation, predetermined amounts of the heteropolysaccharide gum, the homopolysaccharide gum, the cationic crosslinking agent, and the water-soluble base are homogeneously mixed; a wetting agent, such as water, propylene glycol, glycerol, alcohol, or the like, is added thereto to prepare a wet aggregate; and the resulting wet aggregate is dried, and pulverized using a conventional apparatus to prepare granules having a predetermined particle size.
As the lubricant, for example, stearic acid or the like may be used. The mixing of the hydrophobic substance with the controlled release filler may be carried out, for example, by using a liquid in which the hydrophobic substance is dissolved and/or dispersed in an organic solvent, and further granulating the above-mentioned granules together with the liquid.
As the hydrophobic substance, there may be mentioned, for example, a pharmaceutical acceptable hydrophobic cellulose, such as ethyl cellulose or the like.

A combination and a mixing ratio of each component are not particularly limited. In a preferred embodiment, approximately 5 to 50 W/W% of xanthan gum (as the heteropolysaccharide) and locust bean gum (as the homopolysaccharide) (xanthan gum: locust bean gum = approximately 1:1) with respect to the total weight of the pharmaceutical preparation may be contained, and approximately 10 W/W% or less of calcium sulfate (as the water-soluble cationic crosslinking agent) and approximately 20 to 90 W/W% of dextrose (as an inert diluent) may be further contained. To control the release rate, the hydrophobic substance may be added, and, for example, approximately 5 to 10 W/W% of ethyl cellulose may be contained.

A drug-releasing property of the controlled release dosage form of tacrolimus according to the present invention may be evaluated by, for example, a known dissolution test, particularly a dissolution test, method 2 (paddle method), described in the Japanese Pharmacopoeia. In this method, 900 mL of a solution prepared by dissolving 0.005% of hydroxypropyl cellulose (HPC) in the second fluid (JP2) of the disintegration test described in the Japanese Pharmacopoeia is used as a test medium, and a test is carried out at a paddle rotation speed of 100 rpm without the use of a sinker. Samples are collected at predetermined times, and amounts of tacrolimus in the sampling solutions are measured using an HPLC with an ultraviolet and visible detector (a detecting wavelength: 210 nm).

The controlled release dosage form of tacrolimus according to the present invention may be used to reduce an influence caused by the time when tacrolimus is administered to a human. The influence of the administration time can be evaluated by, for example, a maximum blood drug concentration (Cmax). For example, the maximum blood drug concentration in each administration time may be calculated and compared to each other, to evaluate the influence of the administration time. More particularly, the maximum blood drug concentration (a) when administered in the morning, and the maximum blood drug concentration (b) when administered in the evening may be determined, and the ratio (b/a) may be calculated to evaluate the influence of the administration time. When the ratio is close to 1, it may be judged that the influence of the administration time is small.
In the controlled release dosage form of tacrolimus according to the present invention, the ratio (b/a) of the maximum blood drug concentration (b) when administered in the evening to the maximum blood drug concentration (a) when administered in the morning is 0.5 or more and 2.0 or less, preferably 0.7 or more and 1.4 or less. For example, when a pharmaceutical preparation of tacrolimus having a % dissolved of 100% after 4 hours from the beginning of a dissolution test was administered, the ratio (b/a) of the maximum blood drug concentration (b) when administered in the evening to the maximum blood drug concentration (a) when administered in the morning was 0.40. In contrast, when a pharmaceutical preparation of tacrolimus having a % dissolved of 50% after 4 hours from the beginning of a dissolution test was administered, the ratio of the maximum blood drug concentration when administered in the evening to the maximum blood drug concentration when administered in the morning was 0.88, which was significantly higher than that of the above pharmaceutical preparation of tacrolimus having a % dissolved of 100%.

### EXAMPLES

The present invention will now be further illustrated by, but is by no means limited to, the following Examples.

### Examples 1A to 1D: Matrix type pharmaceutical preparation utilizing water-soluble macromolecule (HPMC matrix)

One part of tacrolimus was dissolved in 5 mL of ethanol in a mortar. One part of hydroxypropylmethyl cellulose was added thereto and mixed well in the mortar. Further, 2.5 mL of dichloromethane was added and mixed well until the whole was dissolved. Then, 1 part of croscarmellose sodium and 2 parts of lactose were further added and mixed well in the mortar by using a pestle. The mixture was dried by evaporation until the solvents were completely removed, to obtain a solid dispersion of tacrolimus (hereinafter referred to as solid dispersion 1). In accordance with the formulations shown in Table 1, 5 mg of the solid dispersion 1 (corresponding to 1 mg of tacrolimus), 60 mg of a mixture of lactose and magnesium stearate (St-Mg), and hydroxypropylmethyl cellulose (HPMC) were mixed, and the resulting mixture was compression-molded by using an oil press tabletting machine (tabletting pressure = 1 t/tablet) to obtain the controlled release dosage forms of tacrolimus according to the present invention (1A to 1D).
As the HPMC, TC5S (Shin-Etsu Chemical Co., Ltd.) or 60SH50 (Shin-Etsu Chemical Co., Ltd.) was used.

Evaluation was carried out by a dissolution test, method 2 (paddle method), described in the Japanese Pharmacopoeia. In this method, 900 mL of a solution prepared by dissolving 0.005% of hydroxypropyl cellulose (HPC) in the second fluid (JP2) of the disintegration test described in the Japanese Pharmacopoeia was used as a test medium, and the test was carried out at a paddle rotation speed of 100 rpm without the use of a sinker, at 37°C. Samples were collected at predetermined times, and amounts of tacrolimus in the sampling solutions were measured using an HPLC with an ultraviolet and visible detector (a detecting wavelength = 210 nm).
Each % dissolved after 4 hours is shown in Table 1.

**Table 1**

| Components (unit: mg) | 1A | 1B | 1C | 1D |
|---|---|---|---|---|
| Solid dispersion 1 | 5 | 5 | 5 | 5 |
| Lactose, St-Mg | 60 | 60 | 60 | 60 |
| HPMC (TC5S) | 250 | 335 | - | - |
| HPMC (60SH50) | - | - | 175 | 150 |
| Total weight (mg) | 315 | 315 | 240 | 215 |
| Tablet size (mm) | 9×9R | 9.5×9.5R | 8.5×8.5R | 8.5×8.5R |
| % dissolved | | | | |
| after 4 hours | 44 | 36 | 40 | 43 |

### Examples 2A to 2D: Matrix type pharmaceutical preparation utilizing water-soluble macromolecule (HPMC matrix)

In a mortar, 1 g of Eudragit EPO (degussa; powder product of Eudragit E) was dissolved in 3 mL of ethanol. Further, 200 mg of tacrolimus was added thereto, stirred in the mortar, and mixed well until the whole was dissolved.
The mixture was mixed by stirring until the solvent was completely removed, and dried by evaporation, to obtain a solid dispersion of tacrolimus (solid dispersion 2). In accordance with the formulations shown in Table 2, 6 mg of the solid dispersion 2 (containing a water-insoluble base and the equivalent corresponding to 1 mg of tacrolimus), 60 mg of a mixture of lactose and magnesium stearate (St-Mg), and hydroxypropylmethyl cellulose (HPMC) are mixed, and the resulting mixture is compression-molded by using an oil press tabletting machine (tabletting pressure = 1 t/tablet) to obtain the controlled release dosage forms of tacrolimus according to the present invention (2A to 2D).
As the HPMC, TC5S (Shin-Etsu Chemical Co., Ltd.) or 60SH50 (Shin-Etsu Chemical Co., Ltd.) was used.

**Table 2**

| Components (unit: mg) | 2A | 2B | 2C | 2D |
|---|---|---|---|---|
| Solid dispersion 2 | 6 | 6 | 6 | 6 |
| Lactose, St-Mg | 60 | 60 | 60 | 60 |
| HPMC (TC5S) | 250 | 335 | - | - |
| HPMC (60SH50) | - | - | 175 | 150 |
| Total weight (mg) | 316 | 401 | 241 | 216 |
| Tablet size (mm) | 9×9R | 9.5×9.5R | 8.5×8.5R | 8.5×8.5R |

### Examples 3A to 3D: Matrix type pharmaceutical preparation utilizing water-soluble macromolecule (HPMC + PVP matrix)

In accordance with the formulations shown in Table 3, 5 mg of the solid dispersion 1 (corresponding to 1 mg of tacrolimus) prepared in a similar fashion as shown in Examples 1A to 1D, 60 mg of a mixture of lactose and magnesium stearate (St-Mg), polyvinylpyrrolidone (PVP), and hydroxypropylmethyl cellulose (HPMC) were mixed, and the resulting mixture was compression-molded by using an oil press tabletting machine (tabletting pressure = 1 t/tablet) to obtain the controlled release dosage forms of tacrolimus according to the present invention (3A to 3D).
K90 (Wako Pure Chemical Industries, Ltd.) was used as the PVP, and 90SH100000 (Shin-Etsu Chemical Co., Ltd.) was used as the HPMC.
A dissolution test was carried out by the method for evaluation shown in Examples 1A to 1D. Each % dissolved after 4 hours is shown in Table 3.

**Table 3**

| Components (unit: mg) | 3A | 3B | 3C | 3D |
|---|---|---|---|---|
| Solid dispersion 1 | 5 | 5 | 5 | 5 |
| Lactose, St-Mg | 60 | 60 | 60 | 60 |
| PVP | 200 | 300 | 125 | 133 |
| HPLC | 100 | 100 | 75 | 67 |
| Total weight (mg) | 365 | 465 | 265 | 265 |
| Tablet size (mm) | 9×9R | 10.5×10.5R | 8.5×8.5R | 8.5×8.5R |
| % dissolved | | | | |
| after 4 hours | 43 | 58 | 37 | 50 |

### Examples 4A to 4D: Matrix type pharmaceutical preparation utilizing water-soluble macromolecule (HPMC + PVP matrix)

In accordance with the formulations shown in Table 4, 6 mg of the solid dispersion 2 (containing a water-insoluble base and the equivalent corresponding to 1 mg of tacrolimus) prepared in a similar fashion as shown in Examples 2A to 2D, 60 mg of a mixture of lactose and magnesium stearate (St-Mg), polyvinylpyrrolidone (PVP), and hydroxypropylmethyl cellulose (HPMC) are mixed, and the resulting mixture is compression-molded by using an oil press tabletting machine (tabletting pressure = 1 t/tablet) to obtain the controlled release dosage forms of tacrolimus according to the present invention (4A to 4D).
K90 (Wako Pure Chemical Industries, Ltd.) is used as the PVP, and 90SH100000 (Shin-Etsu Chemical Co., Ltd.) is used as the HPMC.

**Table 4**

| Components (unit: mg) | 4A | 4B | 4C | 4D |
|---|---|---|---|---|
| Solid dispersion 2 | 6 | 6 | 6 | 6 |
| Lactose, St-Mg | 60 | 60 | 60 | 60 |
| PVP | 200 | 300 | 125 | 133 |
| HPMC | 100 | 100 | 75 | 67 |
| Total weight (mg) | 366 | 466 | 266 | 266 |
| Tablet size (mm) | 9×9R | 10.5×10.5R | 8.5×8.5R | 8.5×8.5R |

### Example 5: Controlled release pharmaceutical preparation with a coating membrane

To 5 mg of the solid dispersion 1 (corresponding to 1 mg of tacrolimus) prepared in a similar fashion as shown in Examples 1A to 1D, 60 mg of a mixture of lactose and magnesium stearate (St-Mg) was added, and the resulting mixture was formed into tablets under a tabletting pressure of 40 kg/cm² using a punch having a diameter of 5 mm × 6 mmR, to obtain uncoated tablets having an average weight of 65 mg/tablet. Next, 7 parts of Eudragit RS100 (degussa), 3 parts of Eudragit RL100 (degussa), and 4 parts of polyethylene glycol (PEG400) were added to 50 parts of dichloromethane, and dissolved by stirring, by using a magnetic stirrer, to prepare a coating liquid. The obtained uncoated tablets were immersed in the coating liquid, to obtain the controlled release dosage forms of tacrolimus according to the present invention (5) in which 10.3 wt% of a coating membrane, with respect to the weight of the uncoated tablet, was formed.
Formulations of the uncoated tablet and the coating liquid are shown in Table 5.

A dissolution test was carried out by the method for evaluation shown in Examples 1A to 1D.

**Table 5**

| | 5 |
|---|---|
| [Uncoated tablet] | |
| Solid dispersion 1 | 5 mg |
| Lactose, St-Mg | 60 mg |
| Total weight | 65 mg |
| Tablet size | 5 mm × 6mmR |

| [Coating liquid] | |
|---|---|
| Eudragit RS100 | 1.4 g |
| Eudragit RL100 | 0.6 g |
| PEG400 | 0.8 g |
| Dichloromethane | 25 mL |
| Coating ratio | 10.3% |

### Example 6: Controlled release pharmaceutical preparation with a coating membrane

To 6 mg of the solid dispersion 2 (containing a water-insoluble base and the equivalent corresponding to 1 mg of tacrolimus) prepared in a similar fashion as shown in Examples 2A to 2D, 60 mg of a mixture of lactose and magnesium stearate (St-Mg) is added, and the resulting mixture is formed into tablets under a tabletting pressure of 40 kg/cm² using a punch having a diameter of 5 mm × 6 mmR, to obtain uncoated tablets having an average weight of 66 mg/tablet. Next, 7 parts of Eudragit RS100 (degussa), 3 parts of Eudragit RL100 (degussa), and 4 parts of polyethylene glycol (PEG400) are added to 50 parts of dichloromethane, and dissolved by stirring, by using a magnetic stirrer, to prepare a coating liquid. The obtained uncoated tablets are immersed in the coating liquid, to obtain the controlled release dosage forms of tacrolimus according to the present invention (6) in which 10.3 wt% of a coating membrane, with respect to the weight of the uncoated tablet, is formed.
Formulations of the uncoated tablet and the coating liquid are shown in Table 6.

**Table 6**

| | 6 |
|---|---|
| [Uncoated tablet] | |
| Solid dispersion 2 6 mg | |
| Lactose, St-Mg | 60 mg |
| Total weight | 66 mg |
| Tablet size | 5 mm × 6 mmR |

| [Coating liquid] | |
|---|---|
| Eudragit RS100 | 1.4 g |
| Eudragit RL100 | 0.6 g |
| PEG400 | 0.8 g |
| Dichloromethane | 25 mL |
| Coating ratio | 10.3% |

### Examples 7A to 7D: Multilayered pharmaceutical preparation consisting of drug core and release-controlling layer which are geometrically arranged

### (1) Preparation of granules (A1) which form layer 1 and layer 3 (not containing drug) used in controlling the release of drug

Granules (A1) consisting of the formulation units shown in Table 7 were prepared, and used in preparing the layer 1 and the layer 3 as the top and bottom layers of a three-layered tablet.
In accordance with the formulation shown in Table 7, hydroxypropylmethyl cellulose (HPMC 90SH-15000; Shin-Etsu Chemical Co., Ltd.), hydrogenated caster oil, yellow ferric oxide, and magnesium stearate (St-Mg) were weighed out, and mixed well by using a mortar and pestle until the whole was homogeneously mixed. The resulting homogeneous powder mixture was moistened with an alcohol solution containing 10%(W/V) of ethyl cellulose. The resulting homogeneously wet aggregate was dried at 40°C, and sifted through a screen to obtain granules (A1).

**Table 7**

| | A1 |
|---|---|
| HPMC | 80.25 mg |
| Hydrogenated caster oil | 13.5 mg |
| Yellow ferric oxide | 0.25 mg |
| Ethyl cellulose | 5 mg |
| St-Mg | 1 mg |
| Total | 100.00 mg |

### (2) Preparation of mixed powder (B1) which forms layer 2 containing active substance

In a mortar, 500 mg of tacrolimus was dissolved in 5 mL of ethanol. To the mortar, 500 mg of hydroxypropylmethyl cellulose was added and mixed well. Further, 2.5 mL of dichloromethane was added and mixed well until the whole was dissolved. Then, 1.5 g of lactose was further added and mixed well in the mortar by using a pestle. The mixture was dried by evaporation until the solvents were completely removed, to obtain a solid dispersion of tacrolimus (solid dispersion 3). A mixed powder (B1) consisting of 5 mg of the solid dispersion 3 (corresponding to 1 mg of tacrolimus) and the formulation units shown in Table 8 was prepared, and used in preparing the layer 2 as the intermediate layer of a three-layered tablet.
In accordance with the formulation shown in Table 8, mannitol, microcrystalline cellulose, hydroxypropylmethyl cellulose (HPMC 90SH-15000; Shin-Etsu Chemical Co., Ltd.), and polyvinylpyrrolidone were weighed out, and mixed well by using a mortar and pestle until the whole was homogeneously mixed, to prepare a B1 intermediate powder. To 195 mg of the resulting B1 intermediate powder, 5 mg of the solid dispersion of tacrolimus prepared in Example 2 was added, and the whole was homogeneously mixed well by using a mortar and pestle, to obtain a tacrolimus-containing mixed powder (B1) used as the layer 2 which was the intermediate layer of a three-layered tablet.

**Table 8**

| | B1 intermediate powder |
|---|---|
| Lactose | 11 mg |
| Mannitol | 20 mg |
| HPMC | 20 mg |
| Polyvinylpyrrolidone | 6.4 mg |
| Microcrystalline cellulose | 137.6 mg |
| Total | 195.0 mg |

### (3) Preparation of three-layered tablet (compression molding)

Three-layered tablets were prepared by using an oil press tabletting machine. The tabletting was carried out using a punch having a diameter of 8.0 mm × 8.0 mmR under a tabletting pressure of 1000 kg/punch.
As the layer 3, 150 mg of the granules (A1) prepared in Example 7A to 7D(1) were filled in a die, and a tapping was carried out to flatten the upper surface. As the layer 2, 200 mg of the mixed powder (B1) containing the active substance prepared in Example 7A to 7D(2) was further filled thereon, and a tapping was carried out to flatten the upper surface. Furthermore, as the layer 1, 100 mg of the granules (A1) prepared in Example 7A to 7D(1) were filled thereon. Compression-molding (1000 kg/punch; holding for 10 seconds) was carried out to obtain a three-layered tablet of the present invention (7A) having a tablet weight of 450 mg and containing 1 mg of tacrolimus. The compression-molding may be carried out by filling the layers 1 and 3 in the die in inverse order.

The above procedure was repeated, except that 50 mg of the granules (A1) prepared in Example 7A to 7D(1) as the layers 1 and 3 were used, to obtain a three-layered tablet of the present invention (7B) having a tablet weight of 300 mg and containing 1 mg of tacrolimus.

### (4) Preparation of mixed powder (B2) which forms layer 2 containing active substance

The B1 intermediate powder (125 mg) prepared in Example 7A to 7D(2), 5 mg of the solid dispersion 3, and 10 mg of hydroxypropylmethyl cellulose (HPMC 90SH-15000; Shin-Etsu Chemical Co., Ltd.) were weighed out, and mixed well by using a mortar and pestle until the whole was homogeneously mixed, to prepare a mixed powder (B2) used in forming the layer 2 containing tacrolimus.

### (5) Preparation of two-layered tablet (compression molding)

Two-layered tablets were prepared by using an oil press tabletting machine. The tabletting was carried out using a punch having a diameter of 8.0 mm × 8.0 mmR under a tabletting pressure of 1300 kg/punch. As the layer 1, 150 mg of the granules (A1) prepared in Example 7A to 7D(1) were filled in a die, and a tapping was carried out to flatten the upper surface. As the layer 2, 140 mg of the mixed powder (B2) containing the active substance prepared in Example 7A to 7D(4) was further filled thereon. Compression-molding (1300 kg/punch; holding for 10 seconds) was carried out to obtain a two-layered tablet of the present invention (7C) having a tablet weight of 290 mg and containing 1 mg of tacrolimus. The compression-molding may be carried out by filling the layers 1 and 2 in the die in inverse order.

### (6) Preparation of mixed powder (B3) which forms layer 2 containing active substance

The B1 intermediate powder (180 mg) prepared in Example 7A to 7D(2), 5 mg of the solid dispersion 3, and 10 mg of hydroxypropylmethyl cellulose (HPMC 90SH-15000; Shin-Etsu Chemical Co., Ltd.) were weighed out, and mixed well by using a mortar and pestle until the whole was homogeneously mixed, to prepare a mixed powder (B3) used in forming the layer 2 containing tacrolimus.

### (7) Preparation of two-layered tablet (compression molding)

As the layer 1, 150 mg of the granules (A1) prepared in Example 7A to 7D(1) were filled in a die, and a tapping was carried out to flatten the upper surface. As the layer 2, 195 mg of the mixed powder (B3) containing the active substance prepared in Example 7A to 7D(6) was further filled thereon. Compression-molding (1300 kg/punch; holding for 10 seconds) was carried out to obtain a two-layered tablet of the present invention (7D) having a tablet weight of 345 mg and containing 1 mg of tacrolimus.

### (8) Dissolution test

A dissolution test was carried out by the method for evaluation shown in Examples 1A to 1D. Each % dissolved after 4 hours is shown in Table 9.

**Table 9**

| | 7A | 7B | 7C | 7D |
|---|---|---|---|---|
| % dissolved | | | | |
| after 4 hours | 52.1 | 41.0 | 38.8 | 44.0 |

### (9) Preparation of three-layered tablet containing water-insoluble base in layer 2 (compression molding)

The procedure described in Examples 7A to 7D(2) is repeated, except that 6 mg of the solid dispersion 2 (corresponding to 1 mg of tacrolimus) prepared in Examples 2A to 2D is used as the solid dispersion of tacrolimus, to prepare a mixed powder used as the layer 2. The procedure described in Examples 7A to 7D(3) is repeated, except that the resulting mixed powder is used, to obtain a three-layered tablet containing a water-insoluble base in the layer 2.

### (10) Preparation of two-layered tablet containing water-insoluble base in layer 2 (compression molding)

The procedure described in Examples 7A to 7D(4) is repeated, except that 6 mg of the solid dispersion 2 (corresponding to 1 mg of tacrolimus) prepared in Examples 2A to 2D is used as the solid dispersion of tacrolimus, to prepare a mixed powder used as the layer 2. The procedure described in Examples 7A to 7D(5) is repeated, except that the resulting mixed powder is used, to obtain a three-layered tablet containing a water-insoluble base in the layer 2.

### Examples 8A to 8B: Gel pharmaceutical preparation in which plural gums are combined

Five parts of locust bean gum, 5 parts of xanthan gum, 7 parts of dextrose, and 1 part of calcium sulfate were weighed out, and mixed well by using a mortar and pestle until the whole was homogeneously mixed, to prepare a mixed powder. Then, 2 mL of purified water was divided into two aliquots, and these aliquots were dropwisely added to the resulting mixed powder (1 mL × twice). The whole was mixed well by stirring, by using a pestle, to form granules. The resulting granules were sifted through a 16 mesh (0.59 µm) screen, and dried at a constant temperature of 40°C for 12 hours to obtain a granulated powder (C1).

In accordance with the formulation shown in Table 10, 60 mg of the granulated powder (C1) was mixed with 5 mg of the solid dispersion 1 (corresponding to 1 mg of tacrolimus) prepared in a similar fashion as shown in Examples 1A to 1D, followed by 300 mg of dextrose. The whole was mixed well by stirring, by using a mortar and pestle. The resulting mixture was filled in a die, and compression-molded under tabletting conditions shown in Table 10 by using an oil press tabletting machine, to obtain the tablet of the present invention (8A).

In accordance with the formulation shown in Table 11, 60 mg of the granulated powder (C1) was mixed with 6 mg of the solid dispersion 2 (corresponding to 1 mg of tacrolimus) prepared in a similar fashion as shown in Examples 2A to 2D, followed by 300 mg of dextrose. Compression molding was carried out under tabletting conditions shown in Table 11 by using an oil press tabletting machine, to obtain the tablet of the present invention (8B).

**Table 10**

| Components (unit: mg) | 8A |
|---|---|
| Solid dispersion 1 | 5 |
| Granulated powder | 60 |
| Dextrose | 50 |
| Total weight (mg) | 365 |
| Size (mm) | 9×9R |
| Tabletting pressure (kg/punch) | 250 |

**Table 11**

| Components (unit: mg) | 8B |
|---|---|
| Solid dispersion 2 | 6 |
| Granulated powder | 60 |
| Dextrose | 300 |
| Total weight (mg) | 366 |
| Size (mm) | 9×9R |
| Tabletting pressure (kg/punch) | 250 |

### INDUSTRIAL APPLICABILITY

The present invention can be applied to pharmaceutical preparations.
As above, the present invention was explained with reference to particular embodiments, but modifications and improvements obvious to those skilled in the art are included in the scope of the present invention.

## Claims

1. A controlled release dosage form of tacrolimus, comprising a solid dispersion of tacrolimus, wherein a controlled release base, which is selected from the group consisting of a water-soluble macromolecule, a gum base, and a membrane forming agent and does not form the solid dispersion of tacrolimus, is further contained.

2. The controlled release dosage form of tacrolimus according to claim 1, wherein the form is a tablet.

3. The controlled release dosage form of tacrolimus according to claim 1, comprising the solid dispersion of tacrolimus and the water-soluble macromolecule, wherein a % dissolved after 4 hours from the beginning of a dissolution test is 35% to 85%.

4. The controlled release dosage form of tacrolimus according to claim 3, wherein the water-soluble macromolecule is selected from the group consisting of hydroxypropylmethyl cellulose, hydroxypropylmethyl cellulose phthalate, hydroxymethyl cellulose, methyl cellulose, hydroxyethyl cellulose, hydroxypropyl cellulose, polyvinylpyrrolidone, polyvinyl alcohol, a carboxyvinyl polymer, and carboxymethyl cellulose sodium.

5. The controlled release dosage form of tacrolimus according to claim 1, comprising the solid dispersion of tacrolimus and the gum base, wherein a % dissolved after 4 hours from the beginning of a dissolution test is 35% to 85%.

6. The controlled release dosage form of tacrolimus according to claim 5, wherein the gum base is a combination of a heteropolysaccharide and a homopolysaccharide.

7. The controlled release dosage form of tacrolimus according to claim 6, wherein the heteropolysaccharide is xanthan gum and the homopolysaccharide is locust bean gum.

8. The controlled release dosage form of tacrolimus according to claim 6 or 7, further comprising calcium sulfate and/or a water-soluble base.

9. The controlled release dosage form of tacrolimus according to claim 8, wherein the water-soluble base is selected from the group consisting of dextrose, sucrose, fructose, maltose, xylitol, and citric acid.

10. The controlled release dosage form of tacrolimus according to claim 1, wherein a uncoated tablet or granule containing the solid dispersion of tacrolimus is coated with a coating liquid containing a membrane forming agent, and a % dissolved after 4 hours from the beginning of a dissolution test is 35% to 85%.

11. The controlled release dosage form of tacrolimus according to claim 10, wherein the membrane forming agent is a water-insoluble macromolecule.

12. The controlled release dosage form of tacrolimus according to claim 11, wherein the water-insoluble macromolecule is selected from the group consisting of an aminoalkylmethacrylate copolymer, a methacrylate copolymer, ethyl cellulose, and cellulose acetate.

13. The controlled release dosage form of tacrolimus according to claim 11 or 12, wherein the coating liquid further contains a water-soluble base and/or a water-soluble macromolecule.

14. The controlled release dosage form of tacrolimus according to claim 13, wherein the water-insoluble macromolecule is selected from the group consisting of an aminoalkylmethacrylate copolymer, a methacrylate copolymer, ethyl cellulose, and cellulose acetate, the water-soluble base is selected from the group consisting of dextrose, sucrose, fructose, maltose, xylitol, citric acid, sodium chloride, and polyoxyethylene sorbitan monooleate, and the water-soluble macromolecule is selected from the group consisting of hydroxypropylmethyl cellulose, hydroxypropyl cellulose, polyvinylpyrrolidone, polyethylene glycol, and polyvinyl alcohol.

15. The controlled release dosage form of tacrolimus according to claim 1, comprising
(1) a first layer containing 5% to 90% of the water-soluble macromolecule, and
(2) a second layer containing the solid dispersion of tacrolimus, and the water-soluble macromolecule which does not form the solid dispersion of tacrolimus,
wherein a % dissolved after 4 hours from the beginning of a dissolution test is 35% to 85%.

16. The controlled release dosage form of tacrolimus according to claim 15, further comprising a third layer containing a water-soluble macromolecule.

17. The controlled release dosage form of tacrolimus according to claim 15 or 16, wherein the water-soluble macromolecule is selected from the group consisting of hydroxypropylmethyl cellulose, hydroxypropyl cellulose, hydroxymethyl cellulose, and hydroxyethyl cellulose.

18. The controlled release dosage form of tacrolimus according to any one of claims 15 to 17, further comprising microcrystalline cellulose in the second layer.

19. The controlled release dosage form of tacrolimus according to any one of claims 15 to 18, further comprising a water-soluble base selected from the group consisting of dextrose, sucrose, fructose, maltose, xylitol, citric acid, lactose, and mannitol in the second layer.

20. Use of a controlled release dosage form of tacrolimus for reducing an influence caused by a time when tacrolimus is administered to a human.

21. The use according to claim 20, wherein a ratio (b/a) of a maximum blood drug concentration (b) when administered in the evening to a maximum blood drug concentration (a) when administered in the morning is 0.5 or more.

22. The use according to claim 20 or 21, wherein a % dissolved after 4 hours from the beginning of a dissolution test is 35% to 85%.

23. The use according to any one of claims 20 to 22, comprising the solid dispersion wherein tacrolimus is present in an amorphous state in a solid base composed of ethyl cellulose and hydroxypropylmethyl cellulose.

24. The use according to any one of claims 20 to 23, wherein a solid dispersion of tacrolimus, and a controlled release base which is selected from the group consisting of a water-soluble macromolecule, a gum base, and a membrane forming agent and does not form the solid dispersion of tacrolimus are contained.
